# EUROPEAN PATENT APPLICATION

(11) **EP 4 316 467 A1**
(43) Date of publication of application: **07.02.2024**
(21) Application number: 22781592.5
(22) Date of filing: 30.03.2022
(51) Int. Cl.: A61K 9/127, A61K 9/50, A61K 38/17, A61K 9/00, A61P 35/00, G01N 33/50, G01N 33/68

(54) **FAS-ASSOCIATED FACTOR 1 (FAF1)-LOADED EXOSOMES AND USE THEREOF AS ANTI-CANCER AGENT**

(30) Priority: 30.03.2021 KR 20210040990
(71) Applicant: Kainos Medicine, Inc., Seongnam-si, Gyeonggi-do 13488 (KR)
(72) Inventor: KIM, Eunhee, Seoul 06331 (KR); HAN, Jeong-Hoon, Seongnam-si, Gyeonggi-do 13488 (KR)
(74) Representative: Dehns
(86) International application number: PCT/KR2022/004475
(87) International publication number: WO 2022/211473

(57) **Abstract**

The present invention relates to fas-associated factor 1 (FAF1) protein-loaded exosomes and a cancer treatment use thereof. The present inventors have isolated FAF1 protein-loaded exosomes from HEK 293 cells in which FAF1 having a known tumorsuppressive function is overexpressed, have administered the FAF1 protein-loaded exosomes to a tumor model in which pancreatic cancer cells (MIA PaCa-2), lung cancer cells (A549), colon cancer cells (HCT 116), liver cancer cells (Hep3B), breast cancer cells (MDA-MB-231), kidney cancer cells (Caki-1) and cervical cancer cell (HeLa cell) are transplanted into nude mice through intratumoral injection, and have identified that there were tumor growth inhibitory effects more remarkable than those of a control group not treated with FAF1, and thus the FAF1-loaded exosomes of the present invention can be used as a therapeutic agent for various types of cancer.

## Description

### Technical Field

The present invention relates to FAF1-loaded exosomes, and a use thereof as an anti-cancer agent.

### Background Art

A tumor is a product of the disordered hyperproliferation of abnormal cells, and when such a tumor has destructive proliferative, invasive, and metastatic properties, the tumor is classified as a malignant tumor, that is, cancer. Cancer-related deaths take the first place for causes of death in Korea and are one of the most important causes of death in advanced countries. Until now, in an effort to treat cancer, surgical therapy was developed and used until the 1950s, radiation therapy in the 1960s, and anti-cancer drug therapy in the 1970s. In the 1980s, immunotherapy and gene therapy began to emerge owing to the radical development of basic science, particularly immunology and molecular biology, and many studies are currently ongoing, but it is still insufficient to achieve breakthroughs as existing therapies.

Fas (also known as Fas receptor, CD95, Apo1, or TNFRSF6) is a receptor having the best ability to kill cells among death receptors, and Fas and Fas ligand (Fas L) play an important role in apoptosis. When three Fas ligands bind to three Fas molecules, an adaptor protein called Fas-associated death domain (FADD) binds to a death domain (DD) of Fas. Then, when the death effector domain (DED) of the FADD is combined with the death effector domain (DED) of the inactive caspase-8 (also known as FLICE or MACH), the caspase-8 is activated, and these Fas, FADD, and caspase-8 form a Fas-death-inducing signaling complex (Fas-DISC), and eventually activate the effector caspase that causes cell death, thereby inducing cell death.

Fas-associated factor 1 (FAF1) is a protein associated with the Fas pathway and was found in mice (Chu et al., 1995; Becker et al., 1997) and quails (Frohlich et al., 1998) and humans (Ryu et al., 1999). These have high amino acid homology to each other, and the FAF1 in mice is composed of 649 amino acids, the FAF1 in quails is composed of 648 amino acids, and the FAF1 in humans is composed of 650 amino acids.

In previous studies on FAF1, the FAF1 has been found to have the ability to induce apoptosis, and the minimum region of induction is the 181-381 amino acid region of FAF1. It has been found that FAF1 is a member of Fas-DISC, and DISC is formed by the interaction of the DEDs of caspase-8 and FADD and the 181-381 amino acid region of FAF1, which is structurally similar to the DED (Ryu and Kim, 2003).

It has been reported that the expression level of FAF1 is lowered in various cancers including lung cancer, colon cancer, liver cancer, prostate cancer, brain cancer, and breast cancer (Feng et al., 2017), and the FAF1 protein is attracting attention as a tumor suppressor, and FAF1 is known to suppress tumors via NF-kB inhibition and suppress tumor metastases via TGF-β signals (Park et al., 2004; Park et al., 2007). In addition, it is known that FAF1 inhibits the proliferation of tumors by arresting the cell cycle of tumors at G2/M phase through Aurora-A inhibition (Jang et al., 2008). Korean Patent Laid-open Publication No. 10-2004-0101707 discloses that a fragment of a specific region of FAF1 has activities of inhibiting angiogenesis and tube formation, and cell proliferation, and thus, the fragment can be used as a tumor suppressor.

Extracellular vesicles were first discovered 50 years ago, and since then, it has been found that vesicles exist in all tested biological fluids, and it is known that cell lines *in vitro* release the vesicles in various ranges. Among the extracellular vesicles, exosome is a vesicle composed of a phospholipid bilayer and having a size of 50-200 nm, which is produced in a cell and secreted to the outside, and have been found to play an important role in intercellular communication through intercellular delivery of specific repertoires of nucleic acids (DNA and RNA), proteins, and lipids that are important for homeostasis. For example, exosomes are involved in basic physiological processes such as neurotransmission, antigen presentation, immune response, organ development, and reproductive ability, and also involved in some pathological disorders including cancer progression, cardiovascular disease, inflammation, and prion transmission.

Exosomes were first discovered in the process of releasing and removing intracellular proteins in the final stage of maturation of a red blood cell, leaving only hemoglobin in the red blood cell, and it was observed that these exosomes were released and secreted into the extracellular space, originating from specific compartments in the cell, called multivesicular bodies (MVBs), rather than being directly separated from a plasma membrane, in a study through an electron microscope. That is, when the multivesicular bodies fuse with the plasma membrane, such vesicles are released into the extracellular environment, which is called exosomes.

Many studies are being conducted on what molecular mechanism exosomes are made by, and various types of immune cells including B-lymphocytes, T-lymphocytes, dendritic cells, megakaryocytes, macrophages, and the like, and stem cells and tumor cells are known to produce and secrete exosomes. Although it has been reported that the production of exosomes is dependent or independent on the endosomal sorting complex required for transport (ESCRT), it is difficult to determine the exact mechanism.

Korean Patent Laid-open Publication No. 2004-0015508, as a related art, discloses a method in which a gene for a specific antigen is introduced into a cell line, and a protein of the introduced gene is stably expressed in the cell line and is released into the extracellular space through exosomes, and a method for using the exosomes as a vaccine. In addition, Korean Patent No. 10-2053065 relates to a pH-sensitive exosome composition using hyaluronic acid and doxorubicin, and discloses a method for preparing a pH-sensitive exosome using doxorubicin and a high molecular material chemically combining hyaluronic acid and 3-diethylamino propylamine, and a cancer cell killing effect of the exosome. In addition, Korean Patent Laid-open Publication No. 10-2018-0078173 relates to a novel exosome-based anti-cancer agent, and discloses a recombinant exosome in which a receptor tyrosine kinase and SIRP, which are phagocytosis promoting proteins, are presented on the surface thereof and a recombinant exosome including an asparaginase , a protein toxin, an antibody specific to a cancer antigen or a fragment of the antibody, a tumor suppressor gene or an anti-angiogenic factor, and the like, which are anti-cancer proteins. In addition, Korean Patent Application No. 10-2019-0059724 discloses a method for screening candidate drugs for treating neurodegenerative diseases using confirming that FAF1 is loaded in exosomes and secreted into the extracellular space, but no study results related to cancer cells have been reported.

By using exosomes, cargo materials are protected from the extracellular environment, resulting in longer half-lives and, optionally, entry into the target cell. However, not all proteins are loaded into exosomes as cargo materials, and even when loaded into exosomes, the loaded cargo materials do not enter all cells. Also, it is technically difficult to load largesized high molecular proteins inside an exosome, and the effectiveness of the loaded protein may be significantly deteriorated when the protein is engineered for loading. It has been reported that FAF1 is spontaneously loaded into an exosome in a nerve cell, but whether the FAF1 loaded in the exosome enters the cell and functions properly varies with the type of cell, and there is a limitation in that this must be verified by experiments. Therefore, if it is confirmed that FAF1 delivery using exosomes functions properly in cancer cells and it is possible to mass-produce the FAF1-loaded exosomes, the FAF1-loaded exosomes may be applied as a tumor suppressor in various cancers.

Accordingly, in order to determine whether the FAF1 known as tumor suppression function is loaded into exosomes to deliver the same to various types of tumors and the FAF1 loaded in the exosomes can suppress tumors, the present inventors have isolated exosomes from cells in which FAF1 was overexpressed, checked whether FAF1 was spontaneously loaded in the isolated exosomes, confirmed that cancer cells were treated with the isolated FAF1-loaded exosomes and the colony formation was suppressed, and confirmed the effect of significantly inhibiting tumor growth compared with other control groups including groups treated with recombinant FAF1 proteins when the FAF1 protein-loaded exosomes were administered to various mouse cancer models to which cancer cells were transplanted, thereby completing the present invention.

### Disclosure of the Invention

### Technical Problem

An object of the present invention is to provide a pharmaceutical composition for treating cancer, containing FAF1 protein-loaded exosomes as an active ingredient.

Another object of the present invention is to provide a method for producing FAF1 protein-loaded exosomes.

Still another object of the present invention is to provide a method for screening cancer therapeutic agents having decreased expression of FAF1.

### Solution to Problem

To achieve the above objectives,
the present invention provides a pharmaceutical composition for treating cancer, the composition containing FAF1 protein-loaded exosomes as an active ingredient.

In addition, the present invention provides a method for producing FAF1 protein-loaded exosomes, the method including the steps of: 1) introducing a polynucleotide encoding FAF1 protein into cells to obtain transformed cells; 2) culturing the transformed cells; and 3) isolating exosomes from the cultured cells.

In addition, the present invention provides a method for screening cancer therapeutic agents, the method including the steps of: 1) treating, with a test substance, cancer cells having decreased expression of FAF 1; 2) measuring the level of the FAF1 protein in exosomes isolated from the cells in step 1); and 3) selecting the test substance in which the level of the FAF1 protein in step 2) is increased as compared with a control group which is not treated with the test substance.

### Advantageous Effects of Invention

The present invention relates to Fas-associated factor 1 (FAF1) protein-loaded exosomes and a cancer treatment use thereof. The present inventors have isolated FAF1 protein-loaded exosomes from HEK 293 cells in which FAF1 known to have a tumor suppressive function is overexpressed, and have administered the FAF1 protein-loaded exosomes to a tumor model, in which pancreatic cancer cells (MIA PaCa-2), lung cancer cells (A549), colorectal cancer cells (HCT 116), liver cancer cells (Hep3B), breast cancer cells (MDA-MB-231), kidney cancer cells (Caki-1) and cervical cancer cells (HeLa cells) are transplanted into nude mice, through intratumoral injection, and have confirmed that there were significant tumor growth inhibitory effects as compared with a control group not treated with FAF1, and thus the FAF1-loaded exosomes of the present invention can be used as a therapeutic agent for various types of cancer.

### Brief Description of Drawings

FIG. 1 shows a result of western blotting showing the amount of FAF1 loaded in exosomes isolated using an ultracentrifuge from HEK 293 cells in which FAF1 is overexpressed.
FIG. 2 shows a result of western blotting showing the amount of FAF1 loaded in exosomes isolated using an ultracentrifuge from HeLa cells in which FAF1 is overexpressed.
FIG. 3 shows a result of western blotting showing the amount of FAF1 loaded in exosomes isolated using Exo-quick-TC^{™} from HEK 293 cells in which FAF1 is overexpressed.
FIG. 4 shows a result of western blotting showing the amount of FAF1 loaded in exosomes isolated using Exo-quick-TC^{™} from HeLa cells in which FAF1 is overexpressed.
FIG. 5a shows photographs showing the effect of inhibiting the colony formation when MIA PaCa-2 cells are treated with exosomes isolated from HEK 293 cells, in which FAF1 is overexpressed.
FIG. 5b is a graph showing the number of colonies when MIAPaCa-2 cells are treated with exosomes isolated from HEK 293 cells in which FAF1 is overexpressed.
FIG. 6a shows photographs showing the effect of inhibiting the colony formation when A549 cells are treated with exosomes isolated from HEK 293 cells in which FAF1 is overexpressed.
FIG. 6b is a graph showing the number of colonies when A549 cells are treated with exosomes isolated from HEK 293 cells in which FAF1 is overexpressed.
FIG. 7a shows photographs showing the effect of inhibiting the colony formation when HCT 116 cells are treated with exosomes isolated from HEK 293 cells in which FAF1 is overexpressed.
FIG. 7b is a graph showing the number of colonies when HCT 116 cells are treated with exosomes isolated from HEK 293 cells in which FAF1 is overexpressed.
FIG. 8a shows photographs showing the effect of inhibiting the colony formation when Hep3B cells are treated with exosomes isolated from HEK 293 cells in which FAF1 is overexpressed.
FIG. 8b is a graph showing the number of colonies when Hep3B cells are treated with exosomes isolated from HEK 293 cells in which FAF1 is overexpressed.
FIG. 9a shows photographs showing the effect of inhibiting the colony formation when MDA-MB-231 cells are treated with exosomes isolated from HEK 293 cells in which FAF1 is overexpressed.
FIG. 9b is a graph showing the number of colonies when MDA-MB-231 cells are treated with exosomes isolated from HEK 293 cells in which FAF1 is overexpressed.
FIG. 10a shows photographs showing the effect of inhibiting the colony formation when HeLa cells are treated with exosomes isolated from HEK 293 cells in which FAF1 is overexpressed.
FIG. 10b is a graph showing the number of colonies when HeLa cells are treated with exosomes isolated from HEK 293 cells in which FAF1 is overexpressed.
FIG. 11a is a graph showing changes in tumor volume over time for MOCK, recombinant FAF1 protein, EXO-CON, and EXO-FAF1 groups when exosomes isolated from HEK 293 cells, in which FAF1 is overexpressed, are administered to nude mice xenotransplanted with MIA PaCa-2 cells.
FIG. 11b is a graph showing tumor volumes for MOCK, recombinant FAF1 protein, EXO-CON, and EXO-FAF1 groups when exosomes isolated from HEK 293 cells, in which FAF1 is overexpressed, are administered to nude mice xenotransplanted with MIAPaCa-2 cells.
FIG. 11c shows photographs of tumors for MOCK, recombinant FAF1 protein, EXO-CON, and EXO-FAF1 groups when exosomes isolated from HEK 293 cells, in which FAF1 is overexpressed, are administered to nude mice xenotransplanted with MIA PaCa-2 cells.
FIG. 11d is a graph showing tumor weights for MOCK, recombinant FAF1 protein, EXO-CON and EXO-FAF1 groups when exosomes isolated from HEK 293 cells, in which FAF1 is overexpressed, are administered to nude mice xenotransplanted with MIAPaCa-2 cells.
FIG. 11e shows photographs showing changes in cell morphology of tumor tissue for MOCK, recombinant FAF1 protein, EXO-CON, and EXO-FAF1 groups when exosomes isolated from HEK 293 cells, in which FAF1 is overexpressed, are administered to nude mice xenotransplanted with MIA PaCa-2 cells.
FIG. 12a is a graph showing changes in tumor volume over time for MOCK, recombinant FAF1 protein, EXO-CON, and EXO-FAF1 groups when exosomes isolated from HEK 293 cells, in which FAF1 is overexpressed, are administered to nude mice xenotransplanted with A549 cells.
FIG. 12b is a graph showing tumor volumes for MOCK, recombinant FAF1 protein, EXO-CON and EXO-FAF1 groups when exosomes isolated from HEK 293 cells, in which FAF1 is overexpressed, are administered to nude mice xenotransplanted with A549 cells.
FIG. 12c shows photographs of tumors for MOCK, recombinant FAF1 protein, EXO-CON, and EXO-FAF1 groups when exosomes isolated from HEK 293 cells, in which FAF1 is overexpressed, are administered to nude mice xenotransplanted with A549 cells.
FIG. 12d is a graph showing tumor weights for MOCK, recombinant FAF1 protein, EXO-CON, and EXO-FAF1 groups when exosomes isolated from HEK 293 cells, in which FAF1 is overexpressed, are administered to nude mice xenotransplanted with A549 cells.
FIG. 12e shows photographs showing changes in cell morphology of tumor tissue for MOCK, recombinant FAF1 protein, EXO-CON, and EXO-FAF1 groups when exosomes isolated from HEK 293 cells, in which FAF1 is overexpressed, are administered to nude mice xenotransplanted with A549 cells.
FIG. 13a is a graph showing changes in tumor volume over time for MOCK, recombinant FAF1 protein, EXO-CON, and EXO-FAF1 groups when exosomes isolated from HEK 293 cells, in which FAF1 is overexpressed, are administered to nude mice xenotransplanted with HCT 116 cells.
FIG. 13b is a graph showing tumor volumes for MOCK, recombinant FAF1 protein, EXO-CON, and EXO-FAF1 groups when exosomes isolated from HEK 293 cells, in which FAF1 is overexpressed, are administered to nude mice xenotransplanted with HCT 116 cells.
FIG. 13c shows photographs of tumors for MOCK, recombinant FAF1 protein, EXO-CON, and EXO-FAF1 groups when exosomes isolated from HEK 293 cells, in which FAF1 is overexpressed, are administered to nude mice xenotransplanted with HCT 116 cells.
FIG. 13d is a graph showing tumor weights for each group of MOCK, recombinant FAF1 protein, EXO-CON and EXO-FAF1 when exosomes isolated from HEK 293 cells, in which FAF1 is overexpressed, are administered to nude mice xenotransplanted with HCT 116 cells.
FIG. 13e shows photographs showing changes in cell morphology of tumor tissue for MOCK, recombinant FAF1 protein, EXO-CON, and EXO-FAF1 groups when exosomes isolated from HEK 293 cells, in which FAF1 is overexpressed, are administered to nude mice xenotransplanted with HCT 116 cells.
FIG. 14a is a graph showing changes in tumor volume over time for MOCK, recombinant FAF1 protein, EXO-CON, and EXO-FAF1 groups when exosomes isolated from HEK 293 cells, in which FAF1 is overexpressed, are administered to nude mice xenotransplanted with Hep3B cells.
FIG. 14b is a graph showing tumor volumes for each group of MOCK, recombinant FAF1 protein, EXO-CON and EXO-FAF1 when exosomes isolated from HEK 293 cells, in which FAF1 is overexpressed, are administered to nude mice xenotransplanted with Hep3B cells.
FIG. 14c shows photographs of tumors for MOCK, recombinant FAF1 protein, EXO-CON, and EXO-FAF1 groups when exosomes isolated from HEK 293 cells, in which FAF1 is overexpressed, are administered to nude mice xenotransplanted with Hep3B cells.
FIG. 14d is a graph showing tumor weights for MOCK, recombinant FAF1 protein, EXO-CON, and EXO-FAF1 groups when exosomes isolated from HEK 293 cells, in which FAF1 is overexpressed, are administered to nude mice xenotransplanted with Hep3B cells.
FIG. 14e shows photographs showing changes in cell morphology of tumor tissue for MOCK, recombinant FAF1 protein, EXO-CON, and EXO-FAF1 groups when exosomes isolated from HEK 293 cells, in which FAF1 is overexpressed, are administered to nude mice xenotransplanted with Hep3B cells.
FIG. 15a is a graph showing changes in tumor volume over time for MOCK, recombinant FAF1 protein, EXO-CON, and EXO-FAF1 groups when exosomes isolated from HEK 293 cells, in which FAF1 is overexpressed, are administered to nude mice xenotransplanted with MDA-MB-231 cells.
FIG. 15b is a graph showing tumor volumes for each group of MOCK, recombinant FAF1 protein, EXO-CON and EXO-FAF1 when exosomes isolated from HEK 293 cells, in which FAF1 is overexpressed, are administered to nude mice xenotransplanted with MDA-MB-231 cells.
FIG. 15c shows photographs of tumors for MOCK, recombinant FAF1 protein, EXO-CON, and EXO-FAF1 groups when exosomes isolated from HEK 293 cells, in which FAF1 is overexpressed, are administered to nude mice xenotransplanted with MDA-MB-231 cells.
FIG. 15d is a graph showing tumor weights for MOCK, recombinant FAF1 protein, EXO-CON, and EXO-FAF1 groups when exosomes isolated from HEK 293 cells, in which FAF1 is overexpressed, are administered to nude mice xenotransplanted with MDA-MB-231 cells.
FIG. 15e shows photographs showing changes in cell morphology of tumor tissue for each group of MOCK, recombinant FAF1 protein, EXO-CON, and EXO-FAF1 when exosomes isolated from HEK 293 cells, in which FAF1 is overexpressed, are administered to nude mice xenotransplanted with MDA-MB-231 cells.
FIG. 16a is a graph showing changes in tumor volume over time for MOCK, recombinant FAF1 protein, EXO-CON, and EXO-FAF1 groups when exosomes isolated from HEK 293 cells, in which FAF1 is overexpressed, are administered to nude mice xenotransplanted with Caki-1 cells.
FIG. 16b is a graph showing tumor volumes for MOCK, recombinant FAF1 protein, EXO-CON, and EXO-FAF1 groups when exosomes isolated from HEK 293 cells, in which FAF1 is overexpressed, are administered to nude mice xenotransplanted with Caki-1 cells.
FIG. 16c shows photographs of tumors for MOCK, recombinant FAF1 protein, EXO-CON, and EXO-FAF1 groups when exosomes isolated from HEK 293 cells, in which FAF1 is overexpressed, are administered to nude mice xenotransplanted with Caki-1 cells.
FIG. 16d is a graph showing tumor weights for MOCK, recombinant FAF1 protein, EXO-CON and EXO-FAF1 groups when exosomes isolated from HEK 293 cells, in which FAF1 is overexpressed, are administered to nude mice xenotransplanted with Caki-1 cells.
FIG. 16e shows photographs showing changes in cell morphology of tumor tissue for MOCK, recombinant FAF1 protein, EXO-CON, and EXO-FAF1 groups when exosomes isolated from HEK 293 cells, in which FAF1 is overexpressed, are administered to nude mice xenotransplanted with Caki-1 cells.
FIG. 17a is a graph showing changes in tumor volume over time for MOCK, recombinant FAF1 protein, EXO-CON, and EXO-FAF1 groups when exosomes isolated from HEK 293 cells, in which FAF1 is overexpressed, are administered to nude mice xenotransplanted with HeLa cells.
FIG. 17b is a graph showing tumor volumes for MOCK, recombinant FAF1 protein, EXO-CON, and EXO-FAF1 groups immediately before the enucleation of tumors when exosomes isolated from HEK 293 cells, in which FAF1 is overexpressed, are administered to nude mice xenotransplanted with HeLa cells.
FIG. 17c shows photographs of tumors for MOCK, recombinant FAF1 protein, EXO-CON, and EXO-FAF1 groups when exosomes isolated from HEK 293 cells, in which FAF1 is overexpressed, are administered to nude mice xenotransplanted with HeLa cells.
FIG. 17d is a graph showing tumor weights for the MOCK, Recombinant FAF1 protein, EXO-CON and EXO-FAF1 groups when exosomes isolated from HEK 293 cells, in which FAF1 is overexpressed, are administered to nude mice xenotransplanted with HeLa cells.
FIG. 17e shows photographs showing changes in cell morphology of tumor tissue for MOCK, recombinant FAF1 protein, EXO-CON, and EXO-FAF1 groups when exosomes isolated from HEK 293 cells, in which FAF1 is overexpressed, are administered to nude mice xenotransplanted with HeLa cells.

### Best Mode for Carrying out the Invention

Hereinafter, the present invention will be described in detail.

The present invention provides a pharmaceutical composition for treating cancer, the composition containing FAF1 protein-loaded exosomes as an active ingredient.

The FAF1 protein of the present invention includes an amino acid sequence represented by SEQ ID NO: 1.

FAF1 of the present invention constitutes a Fas-death-inducing signaling complex (Fas-DISC) and induces apoptosis. The FAF1 is known as a protein that activates several pathways and promotes apoptosis.

The FAF1 mediates cell necrosis through JNK-dependent mitochondrial dysfunction, is involved in cell proliferation by suppressing the G2/M phase in a cell cycle by negatively controlling Aurora-A, is involved in an ubiquitin-proteasome pathway by binding to an ubiquitinated protein and a valosin containing protein (VCP) to regulate the degradation of protein, and unnecessary FAF1 is ubiquitinated through Parkin and degraded through the proteasome pathway.

In addition, the FAF1 is involved in various biochemical processes including apoptosis, inflammation, cell proliferation, and protein homeostasis. The FAF1 is a tumor suppressor, which plays a role in suppressing tumors via NF-κB inhibition, and also suppresses tumor metastases via TGF-β signals.

The exosomes of the present invention are small vesicles composed of two layers of phospholipid membrane secreted by cells, and are known to carry out signal transduction between cells, form disease-specific nucleic acids and proteins and release the same into body fluids, and play an important role in intercellular communication through the intercellular transduction of specific repertoires of nucleic acids, proteins, and lipids which are important in homeostasis. For example, the exosomes are involved in basic physiological processes such as neurotransmission, antigen presentation, immune response, organ development, and reproductive ability, and also involved in some pathological disorders including cancer progression, cardiovascular disease, inflammation, and prion transmission.

The exosomes are secreted into the extracellular environment after a late endosome, known as a multivesicular body (MVB) containing intralumenial vesicles (ILVs) fuses with the cell membrane. The multivesicular body may fuse with the cell membrane to release ILVs, whereas the multivesicular body may fuse with lysosome to degrade the contents. The materials present in the exosomes are not similar to the composition of cytoplasmic materials, and the exosomes may optionally contain RNA and proteins.

Although it has been reported that the production of exosomes is dependent or independent on the endosomal sorting complex required for transport (ESCRT), it is difficult to determine the exact mechanism. Cells secrete proteins, in which signal peptides are present, via the endoplasmic reticulum-Golgi complex. The vesicles containing the proteins in which signal peptides are present move toward the cell membrane, fuse with the cell membrane, and release the proteins into the extracellular space. However, proteins without signal peptides can be secreted via an alternative non-classical secretory pathway. When the proteins without signal peptides are secreted, the proteins are secreted either in the absence or presence of vesicles, and although the exact mechanism of the non-vesicular secretory pathway is unknown, some proteins are secreted via membrane pores and ATP-binding cassette transporters. Vesicular secretion is carried out via extracellular vesicles, including exosomes, and is carried out via vesicles with various sizes.

The exosomes may have an average diameter of 50 nm to 300 nm, but the present invention is not limited thereto.

Exosomes are nano-sized extracellular vesicles which are released by most types of cells and contain small RNAs, lipids, and proteins, and have several advantages over currently available synthetic drug delivery means. These advantages include the ability to overcome natural barriers, inherent cell targeting properties, improved permeability, maintenance effects, and biocompatibility. Based on the original function of delivering biological information, the application of exosomes as a therapeutic agent has been attracting attention.

The cancer is a cancer in which the expression of FAF1 is lowered.

The cancer includes, but is not limited to, cervical cancer, pancreatic cancer, liver cancer, lung cancer (including small cell lung carcinoma and non-small cell carcinoma), breast cancer, kidney cancer, colorectal cancer, squamous cell carcinoma of the head and neck, bladder cancer, prostate cancer, gastric cancer, endometrial cancer, brain cancer, ovarian cancer, testicular cancer, head cancer, neck cancer, skin cancer (including melanoma and basal carcinoma), mesothelial lining cancer, esophageal cancer, adrenal cancer, thyroid cancer, bone cancer, glioblastoma, mesothelioma, sarcoma, choriocarcinoma, cutaneous basal cell carcinoma, testicular seminoma, leukemia, and malignant lymphoma. In a preferred aspect, the cancer is pancreatic cancer, non-small cell lung cancer, colorectal cancer, liver cancer, triple negative breast cancer, kidney cancer, or cervical cancer.

The pharmaceutical composition of the present invention may include a carrier, a diluent, an excipient, or a mixture thereof, which are commonly used for pharmaceutical formulations. Any pharmaceutically acceptable carrier may be used as long as it is suitable for delivering the composition in the living body. Specifically, the carrier may be compounds described in Merck Index, 13th ed. Merck & Co. Inc., saline, sterilized water, Ringer's solution, dextrose solution, maltodextrin solution, glycerol, ethanol, or a mixture thereof. In addition, typical additives such as antioxidants, buffers, and bacteriostats may be added, if necessary.

When the pharmaceutical composition is formulated, diluents or excipients such as fillers, extenders, binders, wetting agents, disintegrants, and surfactants, which are commonly used, may be added.

The pharmaceutical composition of the present invention may be formulated into oral preparations or parenteral preparations. The oral preparations may include solid preparations and liquid preparations. The solid preparations may be tablets, pills, powders, granules, capsules, or troches, and the solid preparations may be prepared by adding at least one excipient to the composition. The excipient may be starch, calcium carbonate, sucrose, lactose, gelatin, or a mixture thereof. In addition, the solid preparations may include a lubricant, for example, magnesium stearate, and talc. Meanwhile, the liquid preparations may be suspensions, solutions, emulsions, or syrups. In this case, the liquid preparations may contain excipients such as wetting agents, sweeteners, fragrances, and preservatives.

The parenteral preparations may include injections, suppositories, powders for respiratory inhalation, spray aerosols, powders, creams, and the like. The injections may contain sterilized aqueous solutions, non-aqueous solvents, suspending agents, emulsifiers, and the like. In this case, as the non-aqueous solvents or the suspending agents, propylene glycol, polyethylene glycol, and vegetable oil such as olive oil, or injectable ester such as ethyl oleate, or the like may be used.

The pharmaceutical composition of the present invention may be administered orally or parenterally according to the desired method. Parenteral administration may include, but is not limited to, a method of intrabronchial inhalation, intraperitoneal, intrarectal, subcutaneous, intravenous, intramuscular, intrathoracic, or intratumoral injection. In a preferred aspect, the pharmaceutical composition of the present invention may be injected by the method of intratumoral injection.

The pharmaceutical composition may be administered in a pharmaceutically effective amount. This may vary with a type of disease, severity, drug activity, patient's sensitivity to drug, an administration time, an administration route, duration of treatment, drugs used simultaneously, and the like. However, for a desired effect, the amount of the active ingredient contained in the pharmaceutical composition according to the present invention may be 0.0001-1,000 mg/kg, specifically, 0.001-500 mg/kg. The administration may be performed once to several times a day.

The pharmaceutical composition of the present invention may be administered alone or in combination with other therapeutic agents. In combination administration, the administration may be performed sequentially or simultaneously.

In addition, the present invention provides a method for producing FAF1 protein-loaded exosomes, the method including the steps of:
1) introducing a polynucleotide encoding FAF1 protein into cells to obtain transformed cells;
2) culturing the transformed cells; and
3) isolating exosomes from the cells.

The cells in step 1) are any one or more cells selected from the group consisting of B-lymphocytes, T-lymphocytes, dendritic cells, megakaryocytes, macrophages, stem cells, tumor cells, and HEK293 cells, but are not limited thereto. In a preferred aspect, the cells are HEK 293 cells or HeLa cells.

The transduction may be performed by treating 5 × 10⁶ to 7 × 10⁶ of the cells with 5-10 µg of the 3X Flag-FAF1 plasmid and culturing the cells, and preferably treating 6.5 × 10⁶ of the cells with 8 µg of the 3X Flag-FAF 1 plasmid and culturing the cells.

The exosomes may be isolated by a method commonly used in the art for isolating the exosomes. Size exclusion chromatography, ion exchange chromatography, density gradient centrifugation, differential centrifugation, ultrafiltration, tangential flow filtration, exosome precipitation, total exosome isolation kit, immune-absorbent capture, affinity method, for example, affinity capture, affinity purification, immunoassay, microfluidic separation, or a combination thereof may be performed to isolate exosomes.

According to a specific embodiment of the present invention, the culture medium is collected and centrifuged at 300 x g for 10 minutes, at 2,000 g for 10 minutes, and at 10,000 g for 30 minutes to separate the supernatant, the supernatant is filtered using a 0.2-µm filter, and centrifuged using an ultracentrifuge at 150,000 x g for 70 minutes. The centrifuged supernatant is removed, the pellet is washed by adding PBS, and then centrifuged again for 150,000 x g for 70 minutes to remove the supernatant, thereby isolating exosomes remaining in a lower layer.

The isolated exosomes may be stored at -20 °C and -80 °C.

As used herein, the term "transformation" is a molecular biological technique in which a DNA chain fragment or plasmid etc. having an exogenous gene different from that of the original cell penetrates into the cell and binds to the DNA present in the original cell, thereby altering the genetic character of the cell.

The FAF1-loaded exosomes contain FAF1 proteins therein and serve as carriers for transporting FAF 1 proteins to cancer cells or tissues.

The FAF 1 proteins are secreted into the extracellular space via exosomes.

The extracellular-secreted FAF 1 proteins may induce apoptosis in other cells.

The apoptosis is a kind of programmed cell death that may occur in multicellular organisms. The apoptosis leads to cell death due to changes in cell morphology and biochemical changes inside the cell. This process ends with cell swelling and cracking, cell membrane changes, chromatin condensation and chromosome cleavage, and the cell being engulfed by a phagocyte.

In contrast to necrosis, which is cell death caused by acute cellular injury, apoptosis does not harm organisms but confers advantages on the life cycle thereof. The formation of fingers and toes in the process of differentiation of human embryo is a representative example of apoptosis. In addition, apoptosis is an important mechanism in cell replacement, tissue remodeling, and removal of damaged cells.

Apoptosis occurs in two cases: one in order to eliminate unnecessary parts during the development and differentiation process, and the other in order to protect the other cells when the cell is severely damaged and likely to become cancerous. However, the cells that have problems in the process of apoptosis become cancer cells, and the cancer cells do not die, but rather gradually proliferate and grow through repeated division.

As used herein, the term "culture" refers to a method for growing cells or microorganisms under appropriately artificially controlled environmental conditions. In the present invention, a method for culturing the transformant may be performed using a method widely known in the art.

The medium refers to a known medium used for culturing animal cells, and may be selected from the group of commercially available serum-free media, protein-free media, and chemically defined media.

In addition, the present invention provides a method for screening cancer therapeutic agents, the method including the steps of:
1) treating, with a test substance, cancer cells having decreased expression of FAF1;
2) measuring the level of FAF1 protein in exosomes isolated from the cells in step 1); and
3) selecting the test substance in which the level of FAF1 protein in step 2) is increased as compared with a control group which is not treated with the test substance.

In the method, the cells of step 1) are not limited, but in a preferred aspect, MIA PaCa-2, A549, HCT 116, Hep3B, MDA-MB-231, Caki-1 or HeLa cells.

In the method, the test substance of step 1) means a substance that increases the expression of FAF1, and may be any one selected from the group consisting of a peptide, a protein, a non-peptide compound, a synthetic compound, a fermentation product, a cell extract, a plant extract, and an animal tissue extract, but is not limited thereto.

As a marker for selecting a candidate material used in the present invention, the level of FAF1 protein may be measured using a method known in the art. For example, antibodies that specifically detect the protein are commercially available, and may be used to detect the protein with Western blot, co-immunoprecipitation, immunofluorescence, enzyme-linked immunosorbent assay, and the like. By using such antibodies, the expression level of a specific protein may be specifically confirmed, but the present invention is not limited thereto.

An increase in the expression level of a specific protein may be selected as a candidate substance for a cancer therapeutic agent, as compared with a control group that is not treated with the test substance.

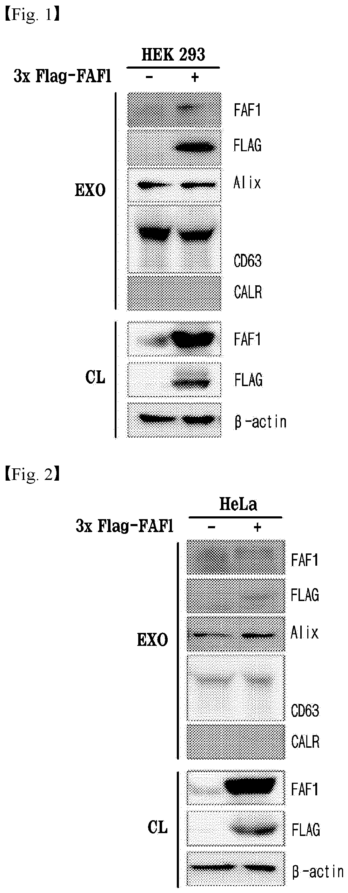

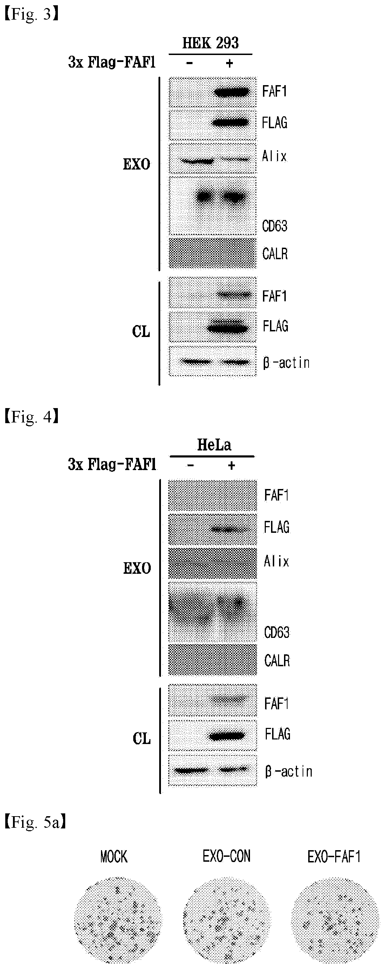

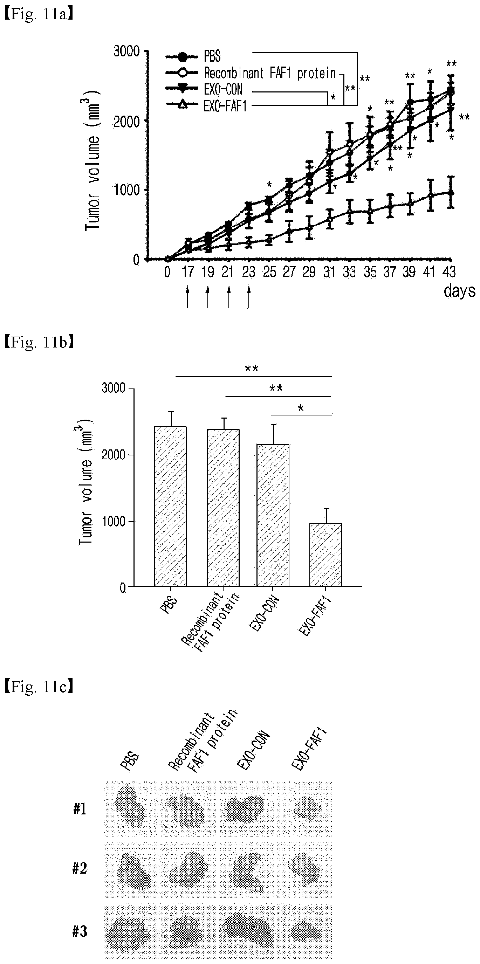

First, it was confirmed through an increase in the amount of FAF1 in the exosomes of HEK 293 cells and HeLa cells, in which FAF1 is overexpressed, that the overexpression of FAF1 in HEK 293 cells and HeLa cells increased the amount of FAF1 contained in the exosomes and made it possible to secrete FAF1 via the exosomes (see FIGS. 1 to 4). FAF1, an apoptosis-promoting factor, is a protein that can inhibit tumor formation. Accordingly, in order to confirm whether exosomes isolated from HEK 293 cells overexpressing FAF1 have the effect of suppressing tumor formation, tumor formation suppression tests were performed on MIAPaCa-2, A549, HCT 116, Hep3B, MDA-MB-231, or HeLa cells, and it was confirmed that the number of colonies formed when exosomes isolated from HEK 293 cells overexpressing FAF1 were treated was smaller than when exosomes isolated from HEK 293 cells not overexpressing FAF1 (see FIGS. 5a, 5b, 6a, 6b, 7a, 7b, 8a, 8b, 9a, 9b, 10a, and 10b). In addition, it was confirmed that the volume and weight of the tumor in the case of the FAF1-loaded exosomes were reduced by the intratumoral injection of exosomes isolated from HEK 293 cells overexpressing FAF1 into nude mice xenotransplanted with MIAPaCa-2, A549, HCT 116, Hep3B, MDA-MB-231, Caki-1, or HeLa cells, and it was confirmed that necrosis of cells occurred due to treatment of FAF1 (see FIGS. 11a to 11e, FIGS. 12a to 12e, FIGS. 13a to 13e, FIGS. 14a to 14e, FIGS. 15a to 15e, FIGS. 16a to 16e, and FIGS. 17a to 17e). The above results demonstrate that the loaded FAF1 can be secreted into the extracellular space via exosomes, the FAF1-loaded exosomes are properly delivered to target cancer cells, and the FAF1 can effectively suppress tumors, which means that the FAF1-loaded exosomes can be used as a cancer therapeutic agent.

Hereinafter, the present invention will be described in detail with reference to the following examples.

However, the following examples are only for illustrating the present invention, and the present invention is not limited thereto.

### Modes for the Invention

### <Example 1> Isolation of Exosomes from HEK 293 Cells Overexpressing FAF1

### <1-1> Cell Culture

HEK 293 cells, which are cell lines derived from human embryonic kidneys, were maintained while being subcultured once every two days under the conditions of 5% CO₂ and 37 °C in Dulbecco's modified Eagle's medium (DMEM, WelGENE, Korea) containing antibiotics, 10% fetal bovine serum (FBS, Atlas Biologicals, USA) and antibiotic-antimycotic (penicillin/streptomycin, GIBCOBRL, USA).

### <1-2> Intracellular Transduction

To overexpress FAF1 in cells, the 3x Flag tag-FAF1 plasmid (8 µg) was transduced into 6.5 ×10⁶ cells.

Specifically, the cells were seeded into a 150-mm culture dish, and then the cells were cultured in the DMEM containing 10% FBS for 24 hours under the conditions of 5% CO₂ and 37 °C. The 3x Flag tag-FAF1 was prepared by the method known previously (Yu et al., 2016). A total of 180 µL of a mixture was prepared at a ratio of DNA:BioT (Bioland scientific, USA) = 1:1.5 using DMEM without FBS and antibiotics, and the mixture was reacted at room temperature for 5 minutes. The mixture was added to the cells cultured for 24 hours, and then the cells were reacted for 24 hours under the conditions of 5% CO₂ and 37 °C.

### <1-3> Exosome Isolation

### <1-3-1> Isolation of Exosomes Using Exo-quick-TC^{™}

Exosomes were isolated from cells transduced with the 3x Flag tag-FAF1 plasmid using Exo-quick-TC^{™} (System Biosciences, USA).

Specifically, 24 hours after the transduction, the cells were treated with DMEM (System Biosciences, USA) treated with 10% exosome-depleted FBS, and cultured for 48 hours. The medium of the cultured cells was collected and centrifuged at 3,000 rpm for 15 minutes to collect the supernatant from which the cell debris was removed. The collected medium (10 mL) was treated with 2 mL of Exo-quick buffer, shaken, and well mixed, so that the ratio of medium:Exo-quick buffer became 5:1. After overnight culture at 4 °C, the process of centrifuging the medium at 1,500 g for 30 minutes was repeated twice to obtain exosomes.

### <1-3-2> Isolation of Exosomes Using Ultracentrifuge

Exosomes were isolated from cells transduced with the 3x Flag tag-FAF1 plasmid using an ultracentrifuge (optima XE-100, BECKMAN Coulter, USA).

Specifically, 24 hours after the transduction, the cells were treated with DMEM without FBS and antibiotics and cultured again for 24 hours. The medium of the cultured cells was collected and centrifuged at 300 g for 10 minutes, 2,000 g for 10 minutes, and 10,000 g for 30 minutes to separate the supernatant, and the supernatant was filtered with a 0.2-µm syringe filter (BioFACT, Korea).

To isolate exosomes for use in *in vitro* and *in vivo* experiments, the supernatant was centrifuged at 150.000 g for 70 minutes with 45Ti rotor in the BECKMAN Coulter optima XE-100 ultracentrifuge, the supernatant was discarded, and the pellet was washed with PBS. The washed PBS was centrifuged again at 150.000 g for 70 minutes, the supernatant was then discarded, and the remaining was collected in an EP tube using PBS.

The isolated exosomes were stored at -20 °C and -80 °C, and thawed at 4 °C before use.

### <Example 2> Isolation of Exosomes from HeLa Cells Overexpressing FAF1

### <2-1> Cell Culture

HeLa cells, which are derived from cervical cancer patients, were maintained while being subcultured once every two days under the conditions of 5% CO₂ and 37 °C in DMEM (WelGENE, Korea) containing antibiotics, 10% FBS (Atlas Biologicals, USA), and antibiotic-antimycotic (penicillin/streptomycin, GIBCOBRL, USA).

### <2-2> Intracellular Transduction

To overexpress FAF1 in HeLa cells, the 3x Flag tag-FAF1 plasmid (8 µg) was transduced into 6.5 ×10⁶ cells.

A specific experimental method is the same as in Example 1-2.

### <2-3> Exosome Isolation

Exosomes were isolated from HeLa cells transduced with the 3x Flag tag-FAF1 plasmid using Exo-quick-TC^{™} or an ultracentrifuge.

A specific experimental method is the same as in Example 1-3.

### <Example 3> Culture of Cancer Cell Lines

MIA PaCa-2 (pancreatic cancer cells), Hep3B (liver cancer cells), MDA-MB-231 (triple negative breast cancer cells), Caki-1 (kidney cancer cells), and HeLa (cervical cancer cells) were cultured in an incubator under the conditions of DMEM, 10% FBS, 1% antibiotic-antimycotic (penicillin/streptomycin, GIBCO BRL, USA), 5% CO₂, and 37 °C, and subcultured once every three days. A549 (non-small cell lung cancer cells) and HCT 116 (colorectal cancer cells) were cultured in an incubator in the RPMI 1640 medium under the conditions of 10% FBS, 1% antibiotic-antimycotic, 5% CO₂, 37 °C and subcultured once every three days.

### <Experimental Example 1> Confirmation of Increase in FAF1 in Exosomes when FAF1 is Overexpressed in HEK 293 Cells and HeLa Cells

In order to find out whether more FAF1 enter exosomes when FAF1 is overexpressed in the HEK 293 cells and the HeLa cells, the expression level of FAF1 in exosomes isolated using the ultracentrifuge and Exo-quick-TC^{™} in the HEK 293 cells and HeLa cells transduced with the 3x Flag tag-FAF10 plasmid (8 µg) of Example 1 was confirmed by western blotting.

Specifically, in order to perform western blotting, 50 µL of a mammalian lysis buffer was added to the pellet from which the supernatant was removed and dissolved on ice for 30 minutes. A western blot kit (Amersham Biosciences, UK) was used to prepare 8-10% acrylamide gel, the dissolved pellet was mixed with 5 µL of SDS sample buffer, boiled for 3 minutes, and then put on the gel in a predetermined amount and applied a voltage of 80-100 V to separate the proteins in the pellet by size. The acrylamide gel containing the separated proteins was placed to overlap a nitrocellulose membrane and a current of 200 mA was applied for 2 hours to transfer the proteins to the nitrocellulose membrane. The nitrocellulose membrane to which the protein was transferred was blocked with nonfat dry milk, the FAF1 antibodies were attached thereto, and then the secondary antibodies were attached thereto, the amount of the FAF1 protein was confirmed by the western blot detection kit (AbFrontier, Korea) and ChemiDoc-It Imaging System (UVP, USA).

When exosomes were isolated using the ultracentrifuge, the expression of FAF1 was slightly increased in the exosomes isolated from HEK 293 cells in which FAF1 was overexpressed, as compared to the exosomes isolated from HEK 293 cells in which FAF 1 was not overexpressed (FIG. 1). In the case of HeLa cells, rarely seen was the expression of FAF1 in the exosomes isolated from cells in which FAF 1 was not overexpressed and cells in which FAF 1 was overexpressed (FIG. 2).

When exosomes were isolated using Exo-quick-TC^{™}, the expression level of FAF1 in the exosomes isolated from cells overexpressing FAF 1 was significantly increased in HEK 293 cells (FIG. 3), and FAF 1 was not seen in the exosomes isolated from cells overexpressing FAF 1 in HeLa cells, but it was confirmed through Flag, which is a tag protein, that the amount of FAF1 was increased (FIG. 4).

The method for isolating exosomes using Exo-quick-TC^{™} is simpler than the method for using an ultracentrifuge, and has the advantage of reducing time, but has the disadvantage of causing contamination during the isolation process. In addition, since the method using the ultracentrifuge may isolate the exosomes having higher purity, the present inventors isolated the exosomes using the ultracentrifuge in the following experimental examples in order to confirm the effect of pure exosomes.

### <Experimental Example 2> Confirmation of in vitro Anti-tumor Effect of Exosomes Isolated from HEK 293 Cells Overexpressing FAF1

By using exosomes isolated from HEK 293 cells overexpressing FAF 1, the ability to inhibit the colony formation was evaluated in the cell lines of pancreatic cancer, non-small cell lung cancer, liver cancer, and cervical cancer.

Specifically, HEK 293 cells transduced with 3x Flag tag-FAF1 (0 or 8 µg) were cultured for 24 hours, cultured in the FBS- and antibiotic-free medium for 24 hours in order to promote exosome secretion, and then the medium was collected and exosomes were isolated using an ultracentrifuge (optima XE-100, BECKMAN Coulter). The isolated exosomes were quantified using a spectrophotometer (MECASYS, Korea). To see the *in vitro* effect of inhibiting the tumor colonies of FAF1-loaded exosomes in MIA PaCa-2, A549, HCT 116, Hep3B, MDA-MB-231 or HeLa cell lines, the MIA PaCa-2, Hep3B or HeLa cells were seeded into 6-well plate at 100 cells, and the A549, HCT116 and MDA-MB-231 cells were seeded at 200 cells. After 24 hours, the cells were treated with exosomes without loading FAF1 (exosome control: EXO-CON) and FAF1-loaded exosomes (EXO-FAF1). In the case of MIA PaCa-2, exosomes were measured by a spectrophotometer (MECASYS, Korea), the cells were treated with 6 µg of the exosomes, and then cultured for 12 days; in the case of A549, exosomes were measured by NTA, the cells were treated with 3×10⁸ exosomes, and then cultured for 10 days; in the case of HCT 116, exosomes were measured by NTA, the cells were treated with 3.16×10⁹ exosomes, and then cultured for 10 days; in the case of Hep3B, exosomes were measured by the spectrophotometer (MECASYS, Korea), the cells were treated with 12 µg of the exosomes, and then cultured for 7 days; in the case of MDA-MB_231, exosomes were measured by NTA, the cells were treated with 8.4×10⁹ exosomes, and then cultured for 12 days; and in the case of HeLa, exosomes were measured by NTA, the cells were treated with 1.65×10¹⁰ exosomes, and then cultured for 10 days. The medium was replaced with a fresh medium once every 3 days, and when the medium was replaced, the cells were treated with exosomes according to the respective concentrations. After completion of the experiments, the number of stained colonies was measured by washing with PBS, treating with 1 mL of methanol for 20 minutes, and treating with 1 mL of the crystal violet solution (SIGMA-ALDRICH, USA) for 5 minutes.

### <2-1> Confirmation of Ability to Inhibit Colony formation in Pancreatic Cancer

In order to verify the ability to inhibit the colony formation of the anti-tumor effects of FAF1-loaded exosomes in pancreatic cancer, MIA PaCa-2 cells, which are the pancreatic cancer cell lines, were treated with the FAF1-loaded exosomes, and then checked.

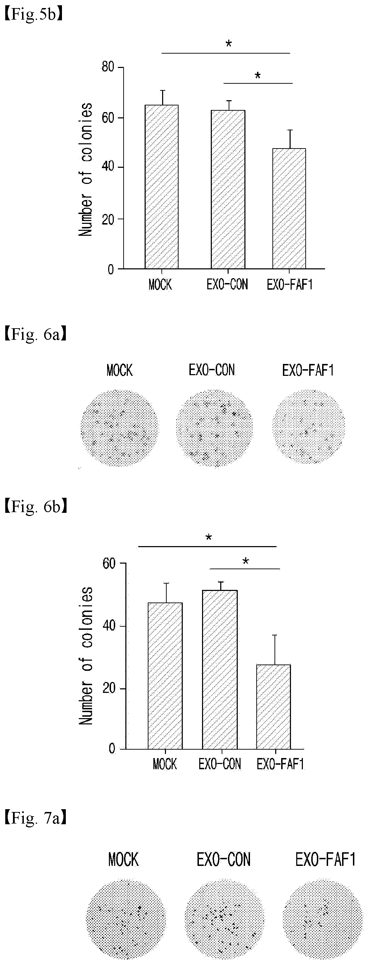

As a result, as shown in FIGS. 5a and 5b, it was confirmed that the least number of colonies were formed in the group treated with EXO-FAF1. By confirming with the figures, it was confirmed that EXO-FAF1 decreased in the colony formation by 24.2±7.27% compared to EXO-CON and by 26.2±7.08% compared to MOCK.

The above results suggest that FAF1-loaded exosomes suppress tumor formation in pancreatic cancer.

### <2-2> Confirmation of Ability to Inhibit Colony formation in Non-Small Cell Lung Cancer

In order to verify the anti-tumor effects of FAF1-loaded exosomes in non-small cell lung cancer, A549 cells, which are non-small cell lung cancer cell lines, were treated with FAF1-loaded exosomes, and then, the ability to inhibit the colony formation was confirmed.

As a result, as shown in FIGS. 6a and 6b, it was confirmed that the least number of colonies were formed in the group treated with EXO-FAF1. It was confirmed that EXO-FAF1 decreased in the colony formation by 46.9±12.88% compared to EXO-CON and by 42.7±13.91% compared to MOCK.

The above results suggest that FAF1-loaded exosomes suppress tumor formation in non-small cell lung cancer.

### <2-3> Confirmation of Ability to Inhibit Colony formation in Colorectal Cancer

In order to verify the anti-tumor effects of FAF1-loaded exosomes in colorectal cancer, HCT 116 cells, which are colorectal cancer cell lines, were treated with FAF1-loaded exosomes, and then, the ability to inhibit the colony formation was confirmed.

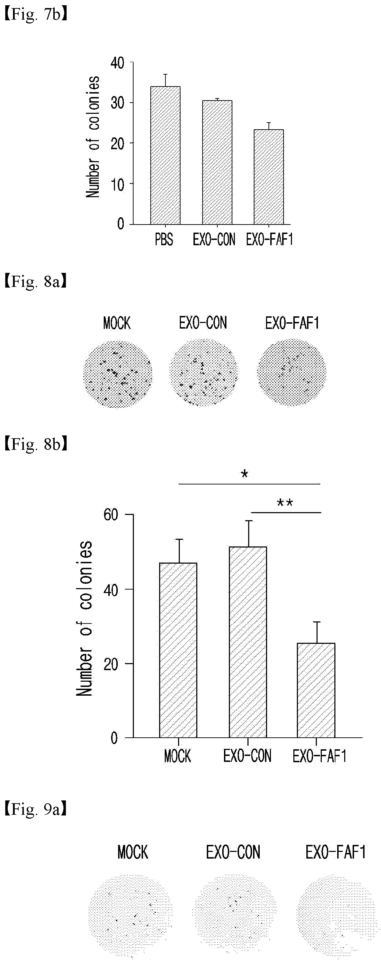

As a result, as shown in FIGS. 7a and 7b, it was confirmed that the least number of colonies were formed in the group treated with EXO-FAF1. It was confirmed that EXO-FAF1 decreased in the colony formation by 23.0±9.07% compared to EXO-CON and by 30.9±8.14% compared to MOCK.

The above results suggest that the FAF1-loaded exosomes suppress tumor formation in colorectal cancer.

### <2-4> Confirmation of Ability to Inhibit Colony formation in Liver Cancer

In order to verify the anti-tumor effects of FAF1-loaded exosomes in liver cancer, Hep3B cells, which are liver cancer cell lines, were treated with FAF1-loaded exosomes, and then, the ability to inhibit the colony formation was confirmed.

As a result, as shown in FIGS. 8a and 8b, it was confirmed that the least number of colonies were formed in the group treated with EXO-FAF1. It was confirmed that EXO-FAF1 decreased in the colony formation by 50.6±10.21% compared to EXO-CON and by 46.1±11.15% compared to MOCK.

The above results suggest that the FAF1-loaded exosomes suppress tumor formation in liver cancer.

### <2-5> Confirmation of Ability to Inhibit Colony formation in Triple Negative Breast Cancer

In order to verify the ability to inhibit the colony formation of the anti-tumor effects of FAF1-loaded exosomes in triple negative breast cancer, MDB-MB-231 cells, which are the triple negative breast cancer cell lines, were treated with the FAF1-loaded exosomes, and then checked.

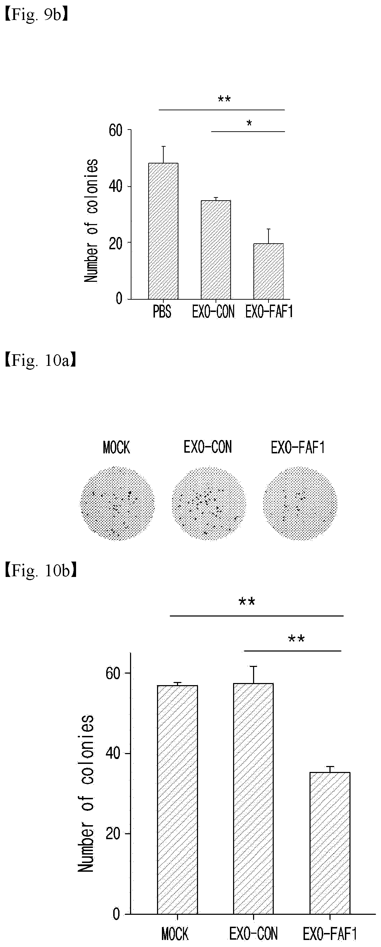

As a result, as shown in FIGS. 9a and 9b, it was confirmed that the least number of colonies were formed in the group treated with EXO-FAF1. By confirming with the figures, it was confirmed that EXO-FAF1 decreased in the colony formation by 43.8±10.40% compared to EXO-CON and by 59.3±7.59% compared to MOCK.

The above results suggest that the FAF1-loaded exosomes suppress tumor formation in triple negative breast cancer.

### <2-6> Confirmation of Ability to Inhibit Colony formation in Cervical Cancer

In order to verify the ability to inhibit the colony formation of the anti-tumor effects of FAF1-loaded exosomes in cervical cancer, HeLa cells, which are the cervical cancer cell lines, were treated with the FAF1-loaded exosomes, and then checked.

As a result, as shown in FIGS. 10a and 10b, it was confirmed that the least number of colonies were formed in the group treated with EXO-FAF1. By confirming with the figures, it was confirmed that EXO-FAF1 decreased in the colony formation by 38.4±6.45% compared to EXO-CON and by 38.0±6.49% compared to MOCK.

The above results suggest that the FAF1-loaded exosomes suppress tumor formation in cervical cancer.

### <Experimental Example 3> Confirmation of in vivo Anti-tumor Effect of Exosomes Isolated from HEK 293 Cells Overexpressing FAF1 in Tumor Xenograft Animal Model

To see the *in vivo* effect of suppressing tumor of exosomes isolated from HEK293 cells overexpressing FAF1, 2-7×10⁶ of each of MIA PaCa-2, A549, HCT 116, Hep3B, MDA-MB-231, Caki-1, and HeLa cell lines were mixed with 100 µL of PBS and 100 µL of matrigel (Corning Life Science, USA), and then xenotransplanted to the dorsal region of an 8-week-old male BALB/cSLC nu/nu mouse (Central Lab. Animal Inc., Korea) through a 26G syringe. Tumor volume was calculated based on (long axis) × (short axis)² × 0.5. When the tumor volume reached 80-140 mm³, PBS, recombinant FAF1 (AngioLab, Korea), EXO-CON, and EXO-FAF1 were injected to mice four times in total once every two days through intratumoral injection, respectively. The tumor volume of the MIA PaCa-2 cell line was measured up to 43 days, the tumor volume of the A549 cell line was measured up to 48 days, the tumor volume of the HCT 116 cell line was measured up to 30 days, the tumor volume of the Hep3B cell line was measured up to 35 days, the tumor volume of the MDA-MB-231 cell line was measured up to 40 days, the tumor volume of the Caki-1 cell line was measured up to 46 days, and the tumor volume of the HeLa cell line was measured up to 41 days. On the last day, after the mice were euthanized, the tumor was enucleated and weighed, and the inside of the tumor was examined through H&E staining.

The extracted tissue for H&E staining was stored in a formalin solution. After 24 hours, dehydration was performed for 20 minutes in 70% ethanol, dehydration was performed twice for 20 minutes in 95% ethanol, and finally, dehydration was performed twice in 100% ethanol for 20 minutes. Then, xylene and paraffin reactions were respectively performed for 3 hours using a tissue processor (Leica, Germany). To cut the tissue sections, the tissue was hardened in paraffin and cut to a thickness of 5 µm using a microtome, and the section was placed on a glass slide. Before staining began, the reaction was performed at 60 °C for 20 minutes using a slide warmer. Next, the reaction was performed in xylene three times for 3 minutes and 30 seconds. After the reaction was performed twice in 100% ethanol, and performed in 95%, 80%, and 70% ethanol in order once, for 3 minutes and 30 seconds each. Then, washing was performed under running water for 3 minutes. Next, the reaction was performed in hematoxylin for 5 minutes, followed by washing for 10 minutes HCl for 4 seconds, 1% ammonia for 10 seconds, washing for 5 minutes, and eosin for 1 minute and 10 seconds in order. Then, the slide was put in 70% ethanol and 85% ethanol in order for 1 minute and 30 seconds, each and then the reaction was performed twice in 100% ethanol for 1 minute and 30 seconds. After the ethanol reaction, a solution in which 100% ethanol and xylene were mixed in a ratio of 1:1 was reacted for 1 minute and 30 seconds. Finally, the reaction was performed with xylene for 3 minutes, and then again with fresh xylene for 4 minutes. The slide was covered with a glass cover using Shandon^{™} Synthetic Mountant (Thermo Scientific, USA). After staining was completed, it was observed through a microscope.

### <3-1> Evaluation of Efficacy in Pancreatic Cancer Xenotransplanted Animal Models

MIA PaCa-2 cells, which are pancreatic cancer cell lines, were xenotransplanted into immunodeficient nude mice to confirm whether FAF1-loaded exosomes have the ability to suppress the tumor formation.

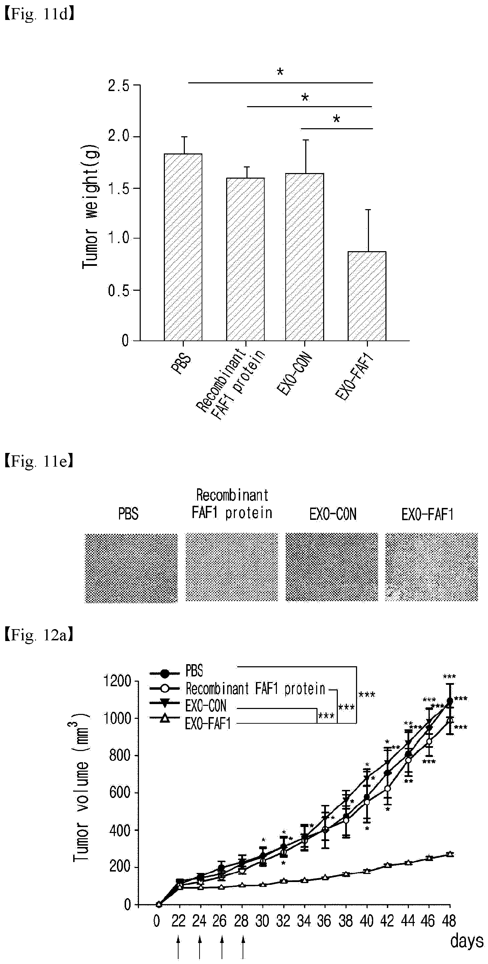

As a result, as shown in FIGS. 11a and 11b, it was confirmed that the tumor volume decreased by 60.46±13.33% in the group treated with FAF1-loaded exosomes compared with the group treated with PBS, by 55.40±15.04% compared with the group treated with EXO-CON, and by 59.71±13.58% compared with the group treated with recombinant FAF1 (AngioLab, Korea). In addition, as shown in FIGS. 11c and 11d, it was confirmed that tumor weight decreased by 52.55±12.13% in the group treated with FAF1-loaded exosomes compared with the group treated with PBS, by 46.74±13.61% compared with the group treated with EXO-CON, and by 45.28±13.98% compared with the group treated with recombinant FAF1 (AngioLab, Korea).

In addition, as a result of observing the cell morphology of the tumor tissue through H&E staining, as shown in FIG. 11e, the cytoplasm size of the tumor tissue of the HEK Exo-FAF1 group was smaller than those of the MOCK group, the recombinant FAF1 group, and the HEK Exo-CON group, and thus it was confirmed that the cell necrosis occurred due to the treatment of HEK Exo-FAF 1.

The above results demonstrate that FAF1 through exosomes also suppresses tumor formation *in vivo.*

### <3-2> Evaluation of Efficacy in Non-small Cell Lung Cancer Xenotransplanted Animal Models

A549 cells, which are non-small cell lung cancer cell lines, were xenotransplanted into immunodeficient nude mice to confirm whether FAF1-loaded exosomes have the ability to suppress the tumor formation.

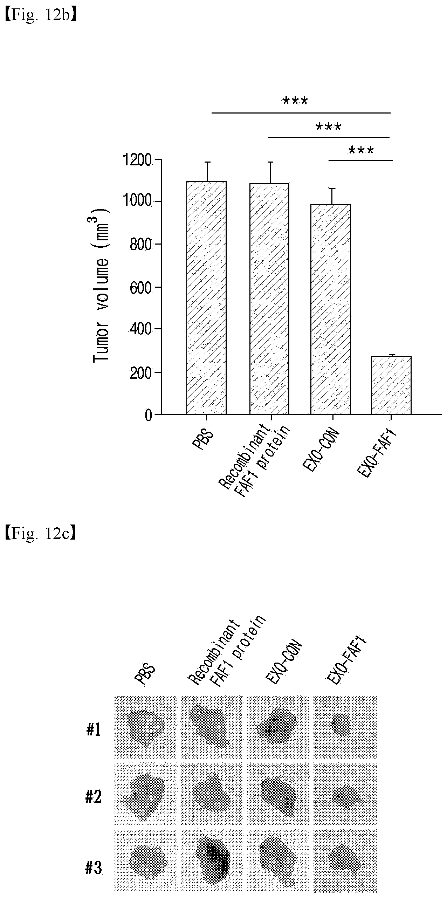

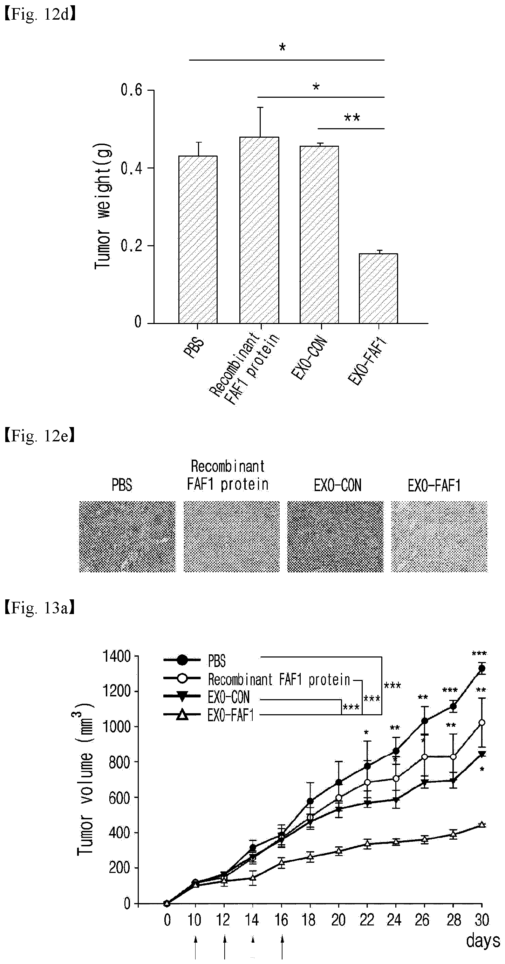

As a result, as shown in FIGS. 12a and 12b, it was confirmed that the tumor volume decreased by 75.45±9.89% in the group treated with FAF1-loaded exosomes compared with the group treated with PBS, by 72.77±10.97% compared with the group treated with EXO-CON, and by 75.14±10.01% compared with the group treated with recombinant FAF1 (AngioLab, Korea). In addition, as shown in FIGS. 12c and 12d, it was confirmed that the tumor weight decreased by 59.07±13.88% in the group treated with FAF1-loaded exosomes compared with the group treated with PBS, by 61.31±13.12% compared with the group treated with EXO-CON, and by 63.19±12.48% compared with the group treated with recombinant FAF1 (AngioLab, Korea). In addition, as shown in FIG. 12e, it was confirmed that necrosis occurred inside the tumor of the group treated with EXO-FAF1.

### <3-3> Evaluation of Efficacy in Colorectal Cancer Xenotransplanted Animal Models

HCT 116 cell lines, which are colorectal cancer cell lines, were xenotransplanted into immunodeficient nude mice to confirm whether FAF1-loaded exosomes have the ability to suppress the tumor formation.

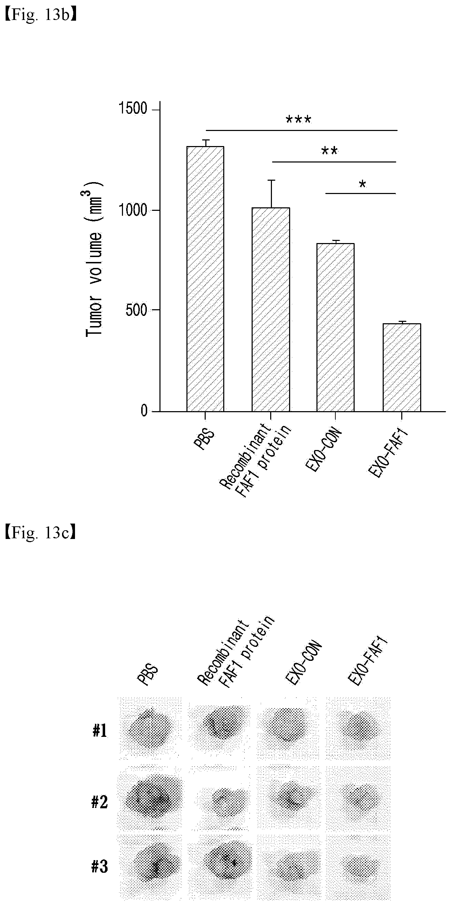

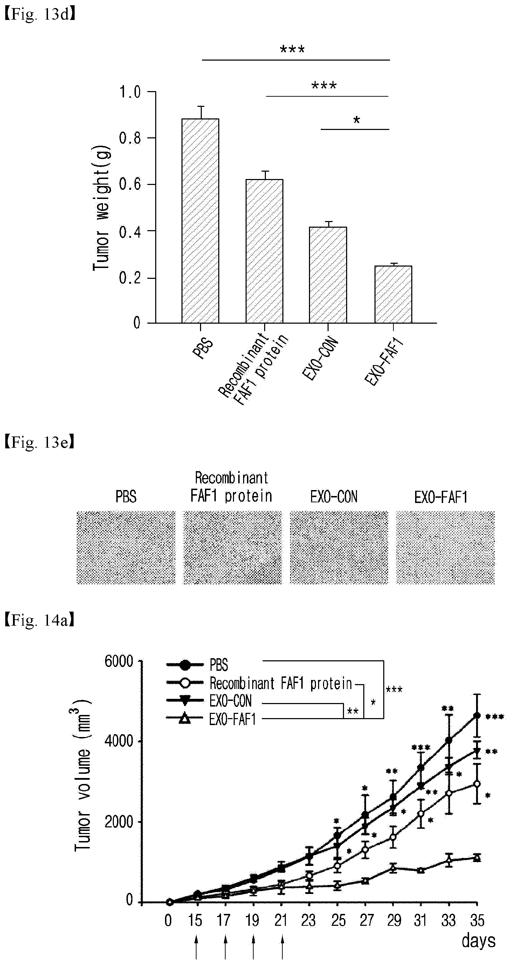

As a result, as shown in FIGS. 13a and 13b, it was confirmed that the tumor volume decreased by 66.87±7.62% in the group treated with FAF1-loaded exosomes compared with the group treated with PBS, by 47.87±12.00% compared with the group treated with EXO-CON, and by 56.93±9.91% compared with the group treated with recombinant FAF1 (AngioLab, Korea). In addition, as shown in FIGS. 13c and 13d, it was confirmed that the tumor weight decreased by 71.88±5.66% in the group treated with FAF1-loaded exosomes compared with the group treated with PBS, by 40.12±12.05% compared with the group treated with EXO-CON, and by 60.09±8.03% compared with the group treated with recombinant FAF1 (AngioLab, Korea). In addition, as shown in FIG. 13e, it was confirmed that necrosis occurred inside the tumor of the group treated with EXO-FAF1.

### <3-4> Evaluation of Efficacy in Liver Cancer Xenotransplanted Animal Models

Hep3B cell lines, which are liver cancer cell lines, were xenotransplanted into immunodeficient nude mice to confirm whether FAF1-loaded exosomes have the ability to suppress the tumor formation.

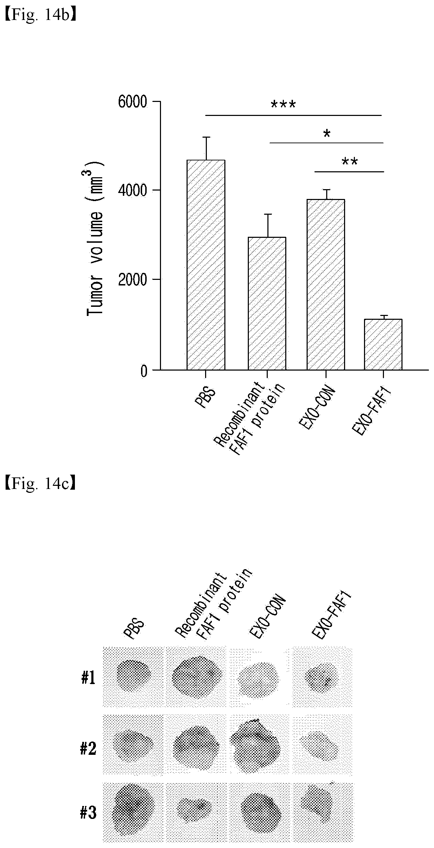

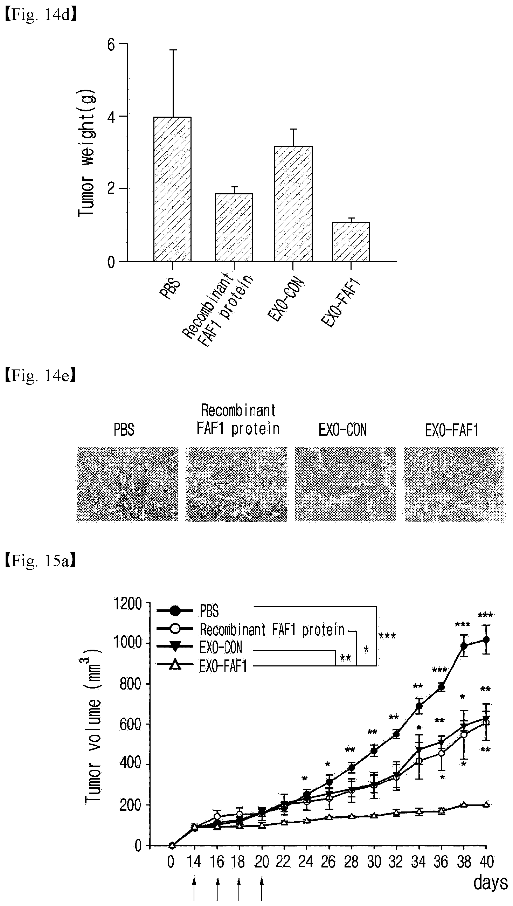

As a result, as shown in FIGS. 14a and 14b, it was confirmed that the tumor volume decreased by 76.16±11.61% in the group treated with FAF1-loaded exosomes compared with the group treated with PBS, by 70.74±14.25% compared with the group treated with EXO-CON, and by 62.44±18.29% compared with the group treated with recombinant FAF1 (AngioLab, Korea). In addition, as shown in FIGS. 14c and 14d, it was confirmed that the tumor weight decreased by 73.48±33.22% in the group treated with FAF1-loaded exosomes compared with the group treated with PBS, by 66.62±42.16% compared with the group treated with EXO-CON, and by 44.44±70.87% compared with the group treated with recombinant FAF1 (AngioLab, Korea). In addition, as shown in FIG. 14e, it was confirmed that necrosis occurred inside the tumor of the group treated with EXO-FAF1.

### <3-5> Evaluation of Efficacy in Triple Negative Breast Cancer Xenotransplanted Animal Models

MDA-MB-231 cell lines, which are triple negative breast cancer cell lines, were xenotransplanted into immunodeficient nude mice to confirm whether FAF1-loaded exosomes have the ability to suppress the tumor formation.

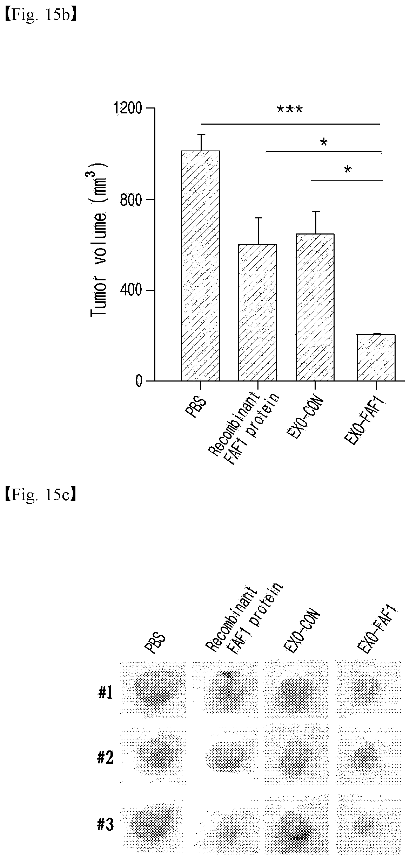

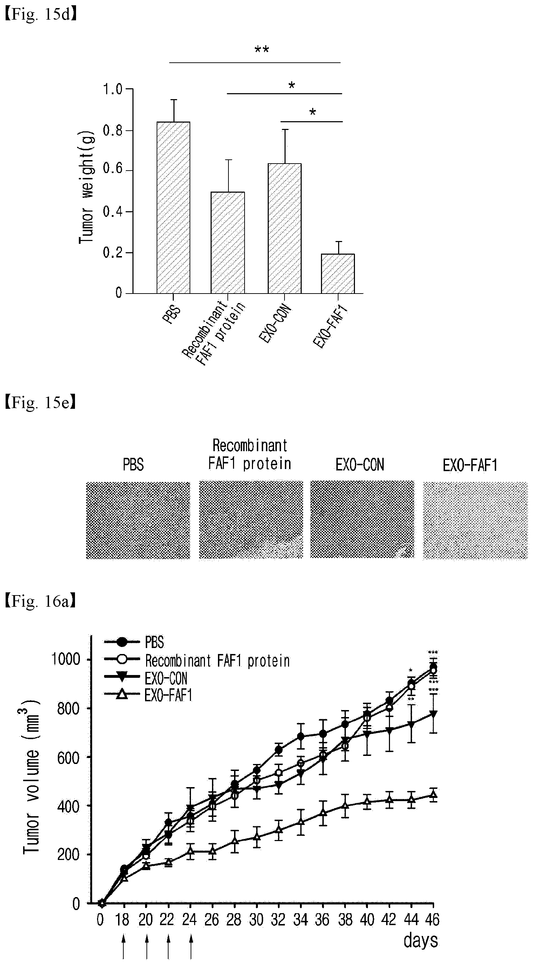

As a result, as shown in FIGS. 15a and 15b, it was confirmed that the tumor volume decreased by 80.38±8.34% in the group treated with FAF1-loaded exosomes compared with the group treated with PBS, by 68.32±13.46% compared with the group treated with EXO-CON, and by 67.22±13.93% compared with the group treated with recombinant FAF1 (AngioLab, Korea). In addition, as shown in FIGS. 15c and 15d, it was confirmed that the tumor weight decreased by 77.03±11.83% in the group treated with FAF1-loaded exosomes compared with the group treated with PBS, by 69.63±15.66% compared with the group treated with EXO-CON, and by 62.50±19.33% compared with the group treated with recombinant FAF1 (AngioLab, Korea). In addition, as shown in FIG. 15e, it was confirmed that necrosis occurred inside the tumor of the group treated with EXO-FAF1.

### <3-6> Evaluation of Efficacy in Kidney Cancer Xenotransplanted Animal Models

Caki-1 cell lines, which are kidney cancer cell lines, were xenotransplanted into immunodeficient nude mice to confirm whether FAF1-loaded exosomes have the ability to suppress the tumor formation.

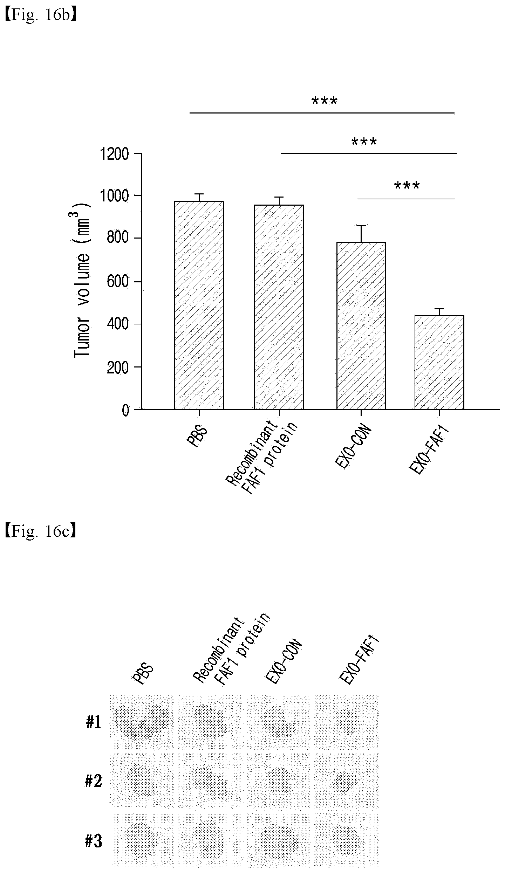

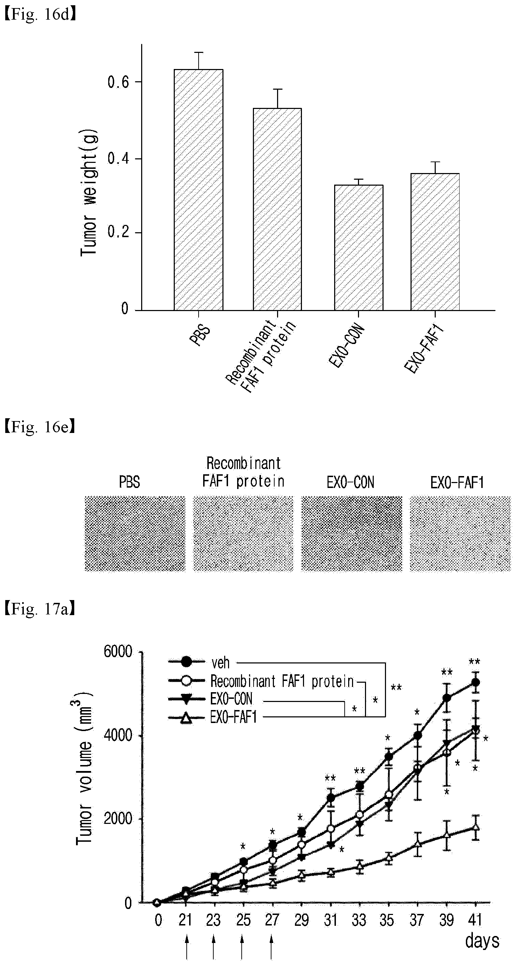

As a result, as shown in FIGS. 16a and 16b, it was confirmed that the tumor volume decreased by 54.23±12.40% in the group treated with FAF1-loaded exosomes compared with the group treated with PBS, by 43.05±15.21% compared with the group treated with EXO-CON, and by 53.56±12.60% compared with the group treated with recombinant FAF1 (AngioLab, Korea). In addition, as shown in FIGS. 16c and 16d, it was confirmed that the tumor weight decreased by 42.11±16.22% in the group treated with FAF1-loaded exosomes compared with the group treated with PBS, by 31.25±19.26% compared with the group treated with recombinant FAF1 (AngioLab, Korea). In addition, as shown in FIG. 16e, it was confirmed that necrosis occurred inside the tumor of the group treated with EXO-FAF1.

### <3-7> Evaluation of Efficacy in Cervical Cancer Xenotransplanted Animal Models

HeLa cells, which are cervical cancer cell lines, were xenotransplanted into immunodeficient nude mice to confirm whether FAF1-loaded exosomes have the ability to suppress the tumor formation.

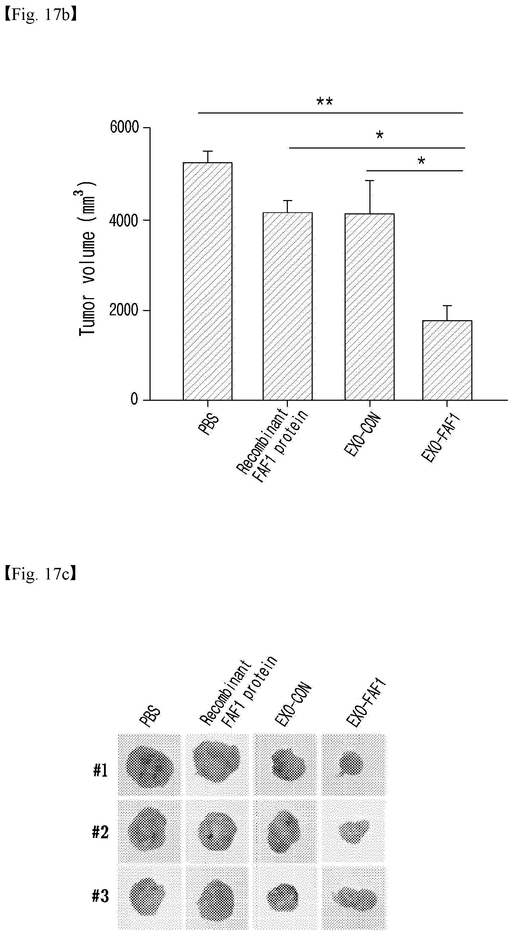

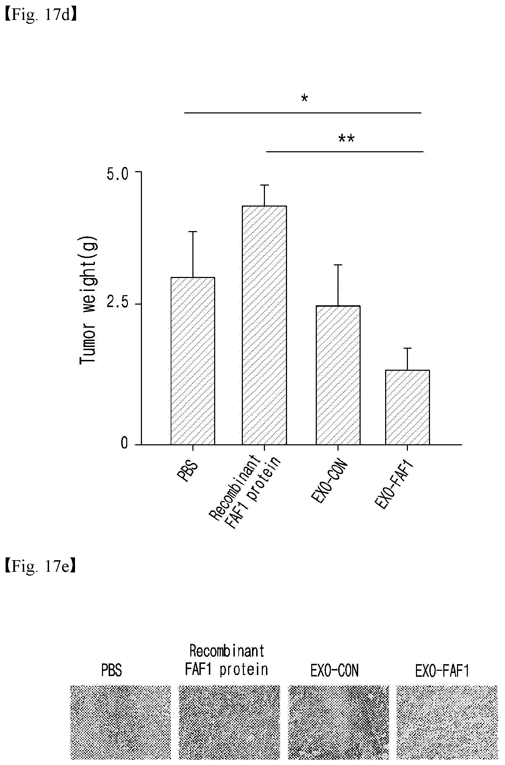

As a result, as shown in FIGS. 17a and 17b, it was confirmed that the tumor volume decreased by 66±11.33% in the group treated with FAF1-loaded exosomes compared with the group treated with PBS, by 56.4±14.52% compared with the group treated with EXO-CON, and by 57.1±14.30% compared with the group treated with recombinant FAF1 (AngioLab, Korea). In addition, as shown in FIGS. 17c and 17d, it was confirmed that the tumor weight decreased by 60±17.59% in the group treated with FAF1-loaded exosomes compared with the group treated with PBS, by 48.6±21.39% compared with the group treated with EXO-CON, and by 70.7±12.17% compared with the group treated with recombinant FAF1 (AngioLab, Korea). In addition, as shown in FIG. 17e, it was confirmed that necrosis occurred inside the tumor of the group treated with EXO-FAF1.

## Claims

1. A pharmaceutical composition for treating cancer, the composition comprising Fas-associated factor 1 (FAF1) protein-loaded exosomes as an active ingredient.

2. The pharmaceutical composition of claim 1, wherein the FAF1 protein comprises an amino acid sequence represented by SEQ ID NO: 1.

3. The pharmaceutical composition of claim 1, wherein the exosomes have a diameter of 50-200 nm.

4. The pharmaceutical composition of claim 1, wherein the FAF1 protein loaded in the exosomes suppresses the tumor formation of other cells.

5. The pharmaceutical composition of claim 1, wherein the cancer is cervical cancer, pancreatic cancer, liver cancer, lung cancer, breast cancer, kidney cancer, colorectal cancer, squamous cell carcinoma of the head and neck, bladder cancer, prostate cancer, gastric cancer, endometrial cancer, brain cancer, ovarian cancer, testicular cancer, head cancer, neck cancer, skin cancer, mesothelial lining cancer, esophageal cancer, adrenal cancer, thyroid cancer, bone cancer, glioblastoma, mesothelioma, sarcoma, choriocarcinoma, cutaneous basal cell carcinoma, testicular seminoma, leukemia, or malignant lymphoma.

6. The pharmaceutical composition of claim 1, wherein the expression of FAF1 is reduced in the cancer.

7. The pharmaceutical composition of claim 1, wherein the exosomes of the composition deliver FAF1 to target tumor cells.

8. The pharmaceutical composition of claim 1, wherein the composition is administered via oral administration, intrabronchial inhalation, or intraperitoneal, intrarectal, subcutaneous, intravenous, intramuscular, intrathoracic, or intratumoral injection.

9. The pharmaceutical composition of claim 1, wherein the composition is administered alone or concurrently and sequentially with other therapeutic agents.

10. The pharmaceutical composition of claim 2, wherein the FAF1 of SEQ ID NO: 1 is loaded in the exosomes.

11. A method for producing FAF1 protein-loaded exosomes, the method comprising the steps of:
1) introducing a polynucleotide encoding FAF1 protein into cells to obtain transformed cells;
2) culturing the transformed cells; and
3) isolating exosomes from the cells.

12. The method of claim 11, wherein the cells in step 1) are any one or more cells selected from the group consisting of B-lymphocytes, T-lymphocytes, dendritic cells, megakaryocytes, macrophages, stem cells and tumor cells, and human embryonic kidney 293 (HEK 293) cells.

13. A method for screening cancer therapeutic agents, the method comprising the steps of:
1) treating, with a test substance, cancer cells having decreased expression of FAF1;
2) measuring the level of FAF1 protein in exosomes isolated from the cells in step 1); and
3) selecting the test substance in which the level of the FAF1 protein in step 2) is increased as compared with a control group which is not treated with the test substance.

14. The method of claim 13, wherein the cells are MIAPaCa-2, MDA-MB-231, HCT 116, Hep3B, Caki-1, A549, or HeLa cells.

15. A method for treating cancer, the method comprising administering FAF1 protein-loaded exosomes to an individual having decreased expression of FAF1.

16. A use of FAF1 protein-loaded exosomes for use in the manufacture of a medicament for treating cancer.
